# EUROPEAN PATENT APPLICATION

(11) **EP 4 643 847 A1**
(43) Date of publication of application: **05.11.2025**
(21) Application number: 23912558.6
(22) Date of filing: 10.11.2023
(51) Int. Cl.: A61K 8/9789, A61K 8/49, A61Q 19/00

(54) **COSMETIC COMPOSITION CONTAINING FERMENTED CARROT LEAF EXTRACT**

(30) Priority: 27.12.2022 KR 20220186241
(71) Applicant: Daebong LS Co., Ltd., Incheon 21697 (KR); UCL Co. Ltd., Jeju-si, Jeju-do 63038 (KR)
(72) Inventor: KIM, Keon-Woo, Incheon 21910 (KR); KIM, Jeong-Mi, Jeju-si, Jeju-do 63137 (KR); MOON, Mi-So, Jeju-si, Jeju-do 63072 (KR); MOON, Ji-Young, Jeju-si, Jeju-do 63098 (KR); PARK, Jin-Oh, Seoul 06276 (KR); YEOM, Hyun-Sook, Jeju-si, Jeju-do 63293 (KR); OH, Tae-Heon, Jeju-si, Jeju-do 63098 (KR); HWANG, Jin, Jeju-si, Jeju-do 63236 (KR); OH, Dae-Ju, Jeju-si, Jeju-do 63278 (KR); LEE, Yoon-Ji, Seogwipo-si, Jeju-do 63608 (KR); MOON, Heon, Jeju-si, Jeju-do 63239 (KR)
(74) Representative: Plasseraud IP
(86) International application number: PCT/KR2023/018056
(87) International publication number: WO 2024/143881

(57) **Abstract**

The present disclosure relates to a cosmetic composition containing a carrot leaf fermentation extract fermented with lactic acid bacteria. A carrot leaf fermentation extract of the present disclosure can provide a cosmetic composition with physiological and nutritional functionality due to its high luteolin content. The cosmetic composition containing the carrot leaf fermentation extract according to the present disclosure can exhibit excellent anti-inflammatory and lipid-suppressing effects.

## Description

### [Technical Field]

The present disclosure relates to a cosmetic composition containing a carrot leaf fermentation extract fermented with lactic acid bacteria.

This application claims priority based on Korean Patent Application No. 10-2022-0186241, filed on December 27, 2022, the entire contents of which are incorporated herein by reference.

### [Background Art]

Carrot leaves are rich in beta-carotene, an immune-boosting ingredient, and anti-stress vitamins A, C, and E, which eliminate active oxygen that destroys human tissues and help relieve stress symptoms that trigger various diseases. Nutritious green and yellow vegetables, which are rich vitamins and minerals, contain various minerals such as potassium, calcium, magnesium, iron, phosphorus, etc. and active ingredients no less than those of general nutritional supplements, and provide enhanced immunity. In addition, they improve constipation due to a rich fiber content and provide positive effects in preventing cardiovascular diseases such as hyperlipidemia, arteriosclerosis, etc. by preventing intestinal uptake of cholesterol, etc. Since carrot leaves are rich in zinc and chromium, which are minerals necessary for the activation of insulin that regulates blood sugar, they are effective in treating diabetic diseases such as postprandial hyperglycemia, etc. In addition, zinc and chlorophyll also suppress gastric inflammation and are effective in treating indigestion.

Regarding such carrot leaves, Korean Patent Publication No. 2017-0001201 discloses a cosmetic composition containing a carrot leaf extract, Korean Patent Publication No. 2022-0125943 discloses a composition for improving blood circulation, anti-inflammation, anti-obesity, and enhancing immune function, which contains carrot leaf powder, and Korean Patent Registration No. 2303467 discloses a composition for hair color treatment, which contains a carrot leaf extract as an ingredient.

However, since it is known that the carrot leaf extract exhibits significantly weakened effects as compared to general chemical substances when it is used as it is, there is a need for a fermentation method, etc. to enhance physiological ingredients of the carrot leaf extract and their activities.

### [References of Related Art]

### [Patent Documents]

(Patent Document 1) Korean Patent Publication No. 10-2017-0001201 (2017.01.04).
(Patent Document 2) Korean Patent Publication No. 10-2022-0125943 (2022.09.15).
(Patent document 3) Korean Patent Registration No. 10-2303467 (2021.09.13).

### [Disclosure]

### [Technical Problem]

The inventors of the present disclosure have conducted researches and made efforts to obtain a fermentation product of carrot leaves with superior physiological and nutritional functionality. As a result, they have completed the present disclosure by discovering that a carrot leaf fermentation extract fermented with specific lactic acid bacteria exhibits excellent anti-inflammatory and lipid-suppressing effects.

Therefore, the present disclosure is directed to providing a cosmetic composition containing a carrot leaf fermentation extract fermented with lactic acid bacteria.

### [Technical Solution]

The present disclosure provides a cosmetic composition containing a carrot leaf fermentation extract fermented with lactic acid bacteria.

The lactic acid bacteria may be one or more strain selected from a group consisting of the genus *Bacillus,* the genus *Streptococcus,* the genus *Lactococcus,* the genus *Enterococcus,* the genus *Lactobacillus,* the genus *Pediococcus,* the genus *Leuconostoc,* the genus *Weissella,* and the genus *Bifidobacterium.*

The lactic acid bacteria may be *Bacillus coagulans* KK7 (KCTC 19023P).

The carrot leaf fermentation extract contains luteolin.

The cosmetic composition may be used for anti-inflammation and lipid suppression purposes.

Meanwhile, the present disclosure provides a method for preparing a carrot leaf fermentation extract, which includes a step of inoculating carrot leaf with *Bacillus coagulans* KK7 (KCTC 19023P) and fermenting the same to obtain a carrot leaf fermentation extract, and a carrot leaf fermentation extract prepared thereby.

The fermentation may be performed at 20 to 60 °C for 1 to 5 days.

### [Advantageous Effects]

A carrot leaf fermentation extract of the present disclosure can provide a cosmetic composition with physiological and nutritional functionality due to its high luteolin content.

The cosmetic composition containing the carrot leaf fermentation extract according to the present disclosure can exhibit excellent anti-inflammatory and lipid-suppressing effects.

### [Brief Description of Drawings]

FIG. 1 is a graph showing the result of measuring the amount of nitric oxide (NO) produced depending on the treatment in an example and a comparative example of the present disclosure.
FIG. 2 is a graph showing the result of measuring lipid content depending on the treatment in an example and a comparative example of the present disclosure.
FIG. 3 shows the images of intracellular lipid droplets (Oil Red O) observed depending on the treatment concentration of a carrot leaf fermentation extract in an example of the present disclosure.

### [Best Mode]

Hereinafter, the present disclosure is described in more detail. Terms or words used in this specification and claims should not be interpreted as limited to their usual or dictionary meanings, and should be interpreted as meanings and concepts that conform to the technical idea of the present disclosure based on the principle that an inventor can appropriately define the concept of terms in order to explain his or her own invention in the best way. Therefore, it should be understood that the configurations described in the exemplary embodiments described in this specification are only the most preferred embodiments of the present disclosure and do not represent all of the technical ideas of the present disclosure, and that there may be various equivalents and modifications that can replace them at the time of filing this application.

The cosmetic composition of the present disclosure contains a carrot leaf fermentation extract fermented with lactic acid bacteria.

The carrot leaf fermentation extract can be obtained by fermenting a carrot leaf extract with lactic acid bacteria or by fermenting raw carrot leaves with lactic acid bacteria.

The carrot leaf extract may be one extracted using water or a C₁-C₄ alcohol as a solvent. But, specifically, it may be one extracted using hot water.

The solvent may be used in an amount 5 to 20 times, specifically 7 to 15 times, the weight of carrot leaves. If the solvent is used in a smaller amount, the active ingredients may not be extracted sufficiently and the extraction yield may decrease. And, if it is used in a larger amount, there is a problem that the extraction process is inefficient.

The extraction may be performed using solvent extraction methods widely known in the art, such as immersion extraction, ultrasonic extraction, and vacuum extraction, although not being limited thereto.

When hot water is used during the extraction, the extraction may be performed at 40 to 100 °C for 0.5 to 24 hours, specifically at 60 to 90 °C for 1 to 12 hours. If the extraction is performed below the above-mentioned limited temperature range, the active ingredients may not be extracted sufficiently.

As the lactic acid bacteria for fermenting the carrot leaf extract, one or more strains selected from a group consisting of the genus *Bacillus,* the genus *Streptococcus,* the genus *Lactococcus,* the genus *Enterococcus,* the genus *Lactobacillus,* the genus *Pediococcus,* the genus *Leuconostoc,* the genus *Weissella,* and the genus *Bifidobacterium,* specifically the genus *Bacillus,* the genus *Lactobacillus,* the genus *Pediococcus,* or the genus *Lactococcus,* more specifically *Bacillus coagulans* KK7 (KCTC 19023P), may be used.

The carrot leaf fermentation extract fermented with the lactic acid bacteria has an increased luteolin content. The fermentation extract may have a luteolin content of 1,000 to 5,000 mg/kg.

Luteolin is contained in many plants including mosses, ferns, conifers, angiosperms, etc., and is rich in dandelion, carrot, red pepper, celery, olive oil, etc. The luteolin is known to affect various biological responses such as antioxidation, anti-inflammation, anti-cancer, neuroprotection, cardioprotection, etc. as an antioxidant and an excellent free radical scavenger, and is also known to inhibit all of enzymatic lipid peroxidation, non-enzymatic lipid peroxidation, and CCl₄-induced lipid peroxidation.

Since the carrot leaf fermentation extract of the present disclosure has a higher luteolin content than general carrot leaf extracts, and may contain about 1,000 to 3,000 mg/kg of luteolin, it can exhibit excellent anti-inflammatory and lipid-suppressing effects.

The cosmetic composition of the present disclosure may contain ingredients commonly used in cosmetic compositions, and may contain conventional auxiliary agents such as antioxidants, stabilizers, solubilizers, vitamins, pigments, and fragrances, as well as carriers.

The cosmetic composition of the present disclosure may be prepared into any formulation commonly prepared in the art. For example, it may be formulated into a solution, a suspension, an emulsion, a paste, a gel, a cream, a lotion, a powder, a soap, a surfactant-containing cleanser, an oil, a powder foundation, an emulsion foundation, a wax foundation, a spray, etc., although not being limited thereto.

More specifically, it may be prepared into the form of a softening lotion, a nourishing lotion, a nourishing cream, a massage cream, an essence, an eye cream, a cleansing cream, a cleansing foam, a cleansing water, a pack, a spray or a powder.

When the formulation of the present disclosure is a paste, cream or gel, animal oil, vegetable oil, wax, paraffin, starch, tragacanth, cellulose derivatives, polyethylene glycol, silicone, bentonite, silica, talc, zinc oxide, etc. may be used as a carrier component.

When the formulation of the present disclosure is a powder or spray, lactose, talc, silica, aluminum hydroxide, calcium silicate or polyamide powder may be used as a carrier component. Particularly, in the case of a spray, a propellant such as chlorofluorohydrocarbon, propane/butane or dimethyl ether may be included additionally.

When the formulation of the present disclosure is a solution or emulsion, a solvent, a solubilizer or an emulsifier is used as a carrier component. For example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan may be used.

When the formulation of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier component.

When the formulation of the present disclosure is a surfactant-containing cleanser, an aliphatic alcohol sulfate, an aliphatic alcohol ether sulfate, a sulfosuccinic acid monoester, an isethionate, an imidazolinium derivative, methyl taurate, sarcosinate, a fatty acid amide ether sulfate, an alkyl amidobetaine, a fatty alcohol, a fatty acid glyceride, a fatty acid diethanolamide, a vegetable oil, a lanolin derivative, an ethoxylated glycerol fatty acid ester, etc. may be used as a carrier component.

When the cosmetic composition of the present disclosure is a soap, surfactant-containing cleanser or surfactant-free cleansing formulation, it may be wiped off, removed or washed off with water after being applied to the skin. As specific examples, the soap may be a liquid soap, a powder soap, a solid soap or an oil soap, the surfactant-containing cleansing formulation may be a cleansing foam, a cleansing water, a cleansing towel or a cleansing pack, and the surfactant-free cleansing formulation may be a cleansing cream, a cleansing lotion, a cleansing water or a cleansing gel, although not being limited thereto.

In addition, the carrot leaf fermentation extract may be included in a pharmaceutical composition for external use on the skin, and the pharmaceutical composition may be in the form of a cream, a gel, a patch, a spray, an ointment, a paste, a lotion, a liniment, a paste or a cataplasm, although not being limited thereto.

Meanwhile, the present disclosure provides a method for preparing a carrot leaf fermentation extract, which includes a step of obtaining a carrot leaf fermentation extract by inoculating carrot leaves with *Bacillus coagulans* KK7 (KCTC 19023P) and fermenting the same.

The inoculation may be made using a carrot leaf extract, and the carrot leaf extract may be specifically a hot water extract, although not being limited thereto. The inoculation may also be made using raw carrot leaves.

The *Bacillus coagulans* KK7 (KCTC 19023P) strain may be inoculated at a concentration of 1 to 10%, specifically 3 to 7%, relative to the weight of the carrot leaf extract or the raw carrot leaves. If the concentration of the inoculated lactic acid bacteria is below 1%, fermentation may not occur sufficiently. And, if it exceeds 10%, over-fermentation may occur.

The carrot leaf extract contains a large amount of carotene, which is a hydrocarbon among carotenoids, and luteolin 7-glucoside, which is a glycoside of glucose and a major intermediate of metabolism as a primary sugar compound in blood. Through the fermentation process, luteolin 7-glucoside, which is a glycoside of glucose, is bioconverted, thereby providing a carrot leaf fermentation extract with a high luteolin content.

The fermentation can be performed at 20 °C to 60 °C for 1 to 5 days, specifically at 30 to 40 °C for 3 to 4 days. If the fermentation occurs below the ranges, fermentation may not occur sufficiently. And, if the fermentation occurs above the ranges, over-fermentation may occur.

Hereinafter, the present disclosure will be described in detail through examples and test examples to specifically explain the present disclosure. However, the examples according to the present disclosure may be modified in various different forms, and the scope of the present disclosure should not be construed as being limited to the examples described below. The examples of the present disclosure are provided to more fully describe the present disclosure to those having ordinary skill in the art.

### [Example] Preparation of carrot leaf fermentation extract using lactic acid bacteria

1 g of a kimchi sample was suspended in sterilized physiological saline and diluted to 10⁻⁶ to prepare a suspension. Then, 100 µL of the suspension was dispensed to a BDTM tryptic soy agar (pancreatic digest of casein 15 g·L⁻¹, papaic digest of soybean meal 5 g·L⁻¹, sodium chloride 5 g·L⁻¹, agar 5 g·L⁻¹; Product No. 236950) medium. An isolated microbial strain was obtained by subculturing a single colony in a medium for 48 hours under dark conditions at 30 ± 1 °C and pH 7.3 ± 0.2, and the strain was finally identified as *Bacillus coagulans* KK7 through 16S-rRNA sequencing. The *Bacillus coagulans* KK7 strain was deposited at the Korean Collection for Type Cultures of the Korea Research Institute of Bioscience and Biotechnology on July 27, 2022, and was assigned the accession number KCTC 19023P.

The isolated strain was subcultured in a tryptic soy agar medium with 24-hour intervals under dark conditions at 30 ± 1 °C and pH 7.3 ± 0.2.

Next, after adding 10 volume equivalents of purified water to dried carrot leaves, an extract from which the carrot leaves were removed was obtained by performing hot water extraction at 80 °C for 60 minutes. After inoculating the cultured *Bacillus coagulans* KK7 strain to the extract at 5.0% of the extract weight and performing fermentation at 35 °C for 3 days, a carrot leaf fermentation extract was obtained by removing the bacteria. After the filtered extract was concentrated under reduced pressure and the moisture was completely removed using a freeze dryer, an extract powder was obtained and used in the experiment.

### [Comparative Example 1] Preparation of carrot leaf extract

After adding 10 volume equivalents of purified water to dried carrot leaves, an extract from which the carrot leaves were removed was obtained by performing hot water extraction at 80 °C for 60 minutes. Then, after concentrating the extract under reduced pressure and completely removing moisture using a freeze dryer, the obtained extract powder was used in the experiment.

### [Test Example 1] Analysis of luteolin content

The physiological ingredients of the extracts of the example and the comparative example were compared. The luteolin content of the extracts of the example and the comparative example was investigated by HPLC analysis using a Shim-pack GIS C18 column. Each of the extracts of the example and the comparative example was precisely weighed to 10 mg, and was dissolved for 30 minutes using an ultrasonic extractor after adding 1 mL of a solution of methanol and dimethyl sulfoxide (6:4, v:v). The luteolin content was then measured after filtering through a syringe filter. A standard was prepared by dissolving in a solution of methanol and dimethyl sulfoxide (6:4, v:v) to a concentration of 1 to 200 µg/mL and filtering with a syringe filter. After the measurement, a peak area was calculated and a standard calibration curve was created using a regression equation. Distilled water containing 0.1% formic acid and acetonitrile were used as a mobile phase, and the flow rate was 1.0 mL/min. A PDA detector (347 nm) was used for detection. Measurement was made for 40 minutes in a gradient mode while changing the proportion of acetonitrile.

**[Table 1]**

| Sample | Luteolin content (mg/kg) | S.D. |
|---|---|---|
| Example | 1933.79 | 4.42 |
| Comparative Example | 441.67 | 1.21 |

As shown in Table 1, the carrot leaf fermentation extract of the example showed a very high luteolin content, confirming that the luteolin content was greatly increased by the fermentation by lactic acid bacteria.

### [Test Example 2] Cell culturing

### [Test Example 2-1] Culturing of macrophages

RAW264.7 macrophages obtained from the American Type Cell Culture Collection (ATCC) were cultured in Dulbecco's modified Eagle's medium (DMEM) containing 100 units/mL of penicillin-streptomycin and 10 vol% of fetal bovine serum (FBS) at 37 °C in a 5% CO₂ incubator. Subculturing was performed at 2-3 day intervals.

### [Test Example 2-2] Culturing of sebocytes

Sebocytes, which are sebaceous gland cells, were provided by Celprogen and cultured in Human Sebocyte Complete Media with Serum, which is a sebocyte-specific medium, in a 5% CO₂ incubator at 37 °C. Subculturing was performed at 7-day intervals.

### [Test Example 3] Evaluation of cytotoxicity

### EZ-cytox assay

The EZ-cytox assay is a representative method for measuring cell viability that utilizes the principle that a water-soluble tetrazolium salt (WST) reacts with the dehydrogenase of living cells to produce an orange-colored, water-soluble formazan.

### [Test Example 3-1] Evaluation of cytotoxicity against macrophages

To examine cytotoxicity, RWA264.7 cells were seeded at 1.0 to 1.5x10⁴ cells/well in a 96-well plate using DMEM medium containing 10% FBS, and cultured under the condition of 37 °C and 5% CO₂ for 18 hours. After exchanging the medium with DMEM containing 10% FBS, the cultured cells were treated with a sample to be evaluated. After that, EZ-cytox was added to each well and reacted under the condition of 37 °C and 5% CO₂ for 30 minutes, and then absorbance was measured at 450 nm using a microplate reader. The average absorbance value for each sample group was calculated, and this value was compared with the absorbance value of the control group to evaluate cell viability.

**[Table 2]**

| | Cytotoxicity | |
|---|---|---|
| | Concentration | RAW264.7 cell growth rate (%) |
| Untreated group | - | 101.5 ± 5.0 |
| Stimulant (LPS) | 1 µg/mL | 100.0 ± 6.0 |
| Example (µg/mL) | 50 | 101.3 ± 3.4 |
| | 100 | 98.8 ± 1.4 |
| | 200 | 99.9 ± 3.7 |
| Comparative Example (µg/mL) | 50 | 100.7 ± 3.8 |
| | 100 | 99.4 ± 1.9 |
| | 200 | 100.2 ± 5.8 |

As shown in Table 2, neither the example nor the comparative example exhibited cytotoxicity against the macrophages.

### [Test Example 3-2] Evaluation of cytotoxicity against sebocytes

To examine cytotoxicity, sebocytes were seeded at 3.0x10⁴ cells/well in a 24-well plate using Human Sebocyte Complete Media with Serum, and cultured under the condition of 37 °C and 5% CO₂ for 48 hours. After exchanging the medium with Human Sebocyte Serum-free Media, the cultured sebocytes were treated with a sample to be evaluated. Afterwards, EZ-cytox was added to each well and reacted under the condition of 37 °C and 5% CO₂ for 30 minutes, and then absorbance was measured at 450 nm using a microplate reader. The average absorbance value for each sample group was calculated, and this value was compared with the absorbance value of the control group to evaluate cell viability.

**[Table 3]**

| | Cytotoxicity | |
|---|---|---|
| | Concentration | Sebocyte cell growth rate (%) |
| Untreated group | - | 100.0 ± 2.2 |
| Example (µg/mL) | 50 | 107.3 ± 2.0 |
| | 100 | 108.0 ± 2.4 |
| | 200 | 112.5 ± 1.9 |
| Comparative Example (µg/mL) | 50 | 95.6 ± 8.8 |
| | 100 | 107.9 ± 4.7 |
| | 200 | 105.8 ± 1.2 |

As shown in Table 3, neither the example nor the comparative example exhibited cytotoxicity against the sebocytes.

### [Test Example 4] Inhibitory activity against nitric oxide (NO) production

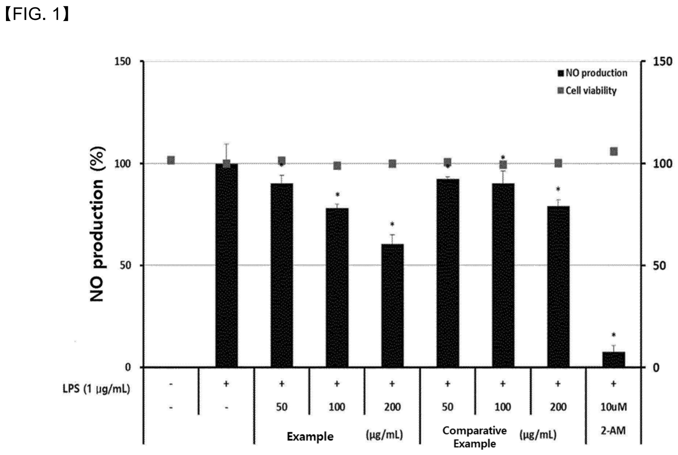

RAW264.7 cells were seeded at 1.5x10⁵ cells/well in a 24-well plate and cultured for 18 hours under the condition of 37 °C and 5% CO₂. The cultured cells were treated with a sample to be evaluated at different concentrations and cultured for 24 hours. 100 µL of a supernatant was collected from the 96-well plate of each test group. Then, after adding 100 µL of a Griess reagent, the mixture was reacted at room temperature for 10 minutes. Afterwards, absorbance was measured at 540 nm using a microplate reader. A control group was treated with 2-amino-4-methylpyridine at a concentration of 2 µM. The NO production depending on the treatment with the example and the comparative example relative to the NO production by a stimulant (LPS) is shown in FIG. 1.

As shown in FIG. 1, it was confirmed that the example suppressed NO production more than the comparative example.

### [Test Example 5] Confirmation of sebum secretion-controlling effect

### [Test Example 5-1] Measurement of lipid content

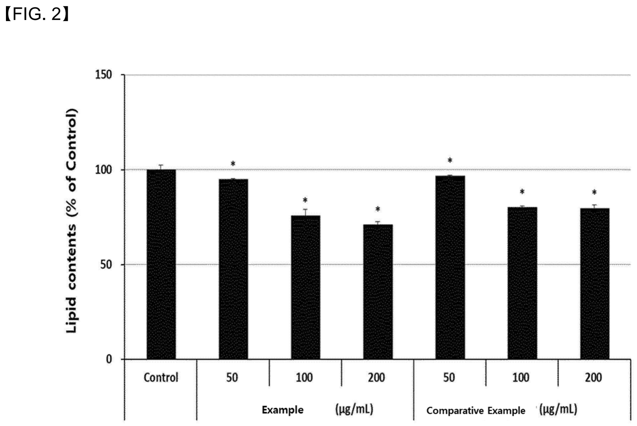

Sebocytes were seeded at 3.0x10⁴ cells/well in a 24-well plate and cultured for 48 hours under the condition of 37 °C and 5% CO₂. After exchanging the medium with Human Sebocyte Serum-free Media, the cells were treated with a sample to be evaluated and cultured for 5 days. Afterwards, the medium was removed for each group. Then, after washing with PBS, lipid content was measured using a lipid extraction kit (Abcam) and a lipid assay kit (Abcam) according to the method provided by the manufacturer. The result of measuring the lipid content depending on the treatment with the example and the comparative example is shown in FIG. 2.

As shown in FIG. 2, it was confirmed that the example suppressed lipid content more than the comparative example.

### [Test Example 5-2] Observation of lipid droplets

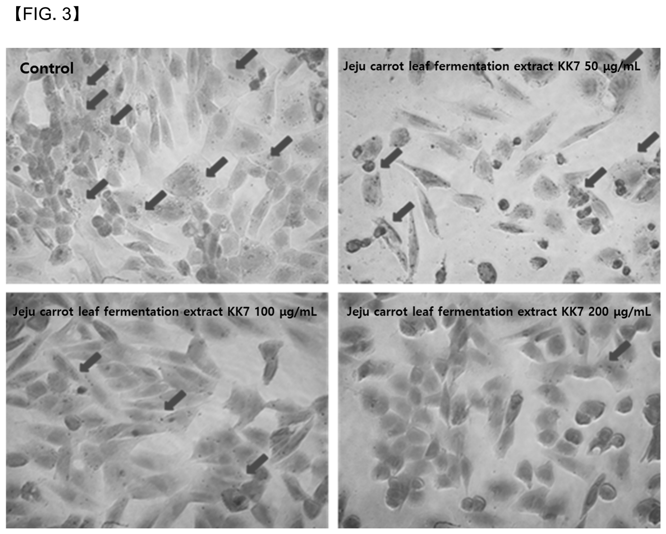

Sebocytes were seeded at 3.0x10⁴ cells/well in a 24-well plate and cultured for 48 hours under the condition of 37 °C and 5% CO₂. After exchanging the medium with Human Sebocyte Serum-free Media, the cells were treated with a sample to be evaluated and cultured for 5 days. Afterwards, the medium was removed for each group. Then, after washing with PBS, lipid droplets were observed using Oil Red O (lipid stain kit) (Abcam) according to the method provided by the manufacturer. FIG. 3 shows the images showing the decrease of lipid droplets depending on the treatment with the example.

As shown in FIG. 3, it was confirmed that the lipid droplets are reduced by the example.

## Claims

1. A cosmetic composition comprising a carrot leaf fermentation extract fermented with lactic acid bacteria.

2. The cosmetic composition according to claim 1, wherein the lactic acid bacteria are one or more strain selected from a group consisting of the genus *Bacillus,* the genus *Streptococcus,* the genus *Lactococcus,* the genus *Enterococcus,* the genus *Lactobacillus,* the genus *Pediococcus,* the genus *Leuconostoc,* the genus *Weissella,* and the genus *Bifidobacterium.*

3. The cosmetic composition according to claim 2, wherein the lactic acid bacteria are *Bacillus coagulans* KK7 (KCTC 19023P).

4. The cosmetic composition according to claim 1, wherein the carrot leaf fermentation extract comprises luteolin.

5. The cosmetic composition according to claim 1, wherein the composition has superior anti-inflammatory and lipid-suppressing effects.

6. A method for preparing a carrot leaf fermentation extract, comprising a step of obtaining a carrot leaf fermentation extract by inoculating carrot leaves with *Bacillus coagulans* KK7 (KCTC 19023P) and fermenting the same.

7. The method for preparing a carrot leaf fermentation extract according to claim 6, wherein the fermentation is performed at 20 to 60 °C for 1 to 5 days.

8. A carrot leaf fermentation extract prepared by the preparation method according to claim 6.
